# EUROPEAN PATENT APPLICATION

(11) **EP 0 525 570 A2**
(43) Date of publication of application: **03.02.1993**
(21) Application number: 92112358.4
(22) Date of filing: 20.07.1992
(51) Int. Cl.: C07K 15/00, C07K 3/02, A61K 39/395

(54) **Anti-idiotypic antibodies that mimic TNF**

(30) Priority: 31.07.1991 US 738631
(71) Applicant: Bayer Corporation, Pittsburgh, PA 15219-2502 (US)
(72) Inventor: Galloway, Cynthia J., Richmond, California 94804 (US); Madanat, Melanie S., San Francisco, California 94122 (US); Dumas, Michael L., Walnut Creek, California 94598 (US)
(74) Representative: Dänner, Klaus, Dr.

(57) **Abstract**

Anti-idiotypic antibodies that mimic tumor necrosis factor (TNF) activity. The antibodies may be raised in one species (e.g. a rabbit) using a monoclonal anti-TNF antibody from another species (e.g. a mouse) as an immunogen. Such anti-idiotypic antibodies were cytotoxic to TNF-sensitive cell lines. The cytotoxic activity of the anti-idiotypic antibodies can be neutralized by antibodies that bind to TNF. The anti-idiotypic antibodies may be useful themselves as TNT antagonists or as TNF agonists, and may also be the basis for a new group of TNF agonists or antagonists.

## Description

### Background of the Invention

Field: This disclosure is concerned generally with making and using antibodies that bind to specific portions of other antibodies and specifically with the use of such antibodies to mimic the biochemical action of a non-antibody substance.

Prior Art: Tumor necrosis factor or TNF mediates a large variety of physiological responses including cytotoxicity, cachexia and septic shock. All the actions of TNF are mediated by binding to TNF receptors present on the surfaces of most cells (reviewed in Vilcek and Lee, J. Biol. Chem. 266: 7313-7316, 1991). It is thought that the deleterious actions of TNF may be ameliorated by substances which prevent binding of TNF to its cellular receptor. Conversely, the beneficial actions of TNF, for example in wound healing (Beutler and Cerami, New Eng. J. Med. 316: 379-85, 1987), may be promoted by substances which stimulate TNF receptors.

Anti-TNF receptor antibodies have been used as substances which both stimulate and inhibit TNF receptor action. Recently, several anti-TNF receptor antibodies have been generated (Engelman et al, J. Biol. Chem. 265: 14497-14504, 1990 and Shalaby et al, J. Exp. Med. 172: 1517-1520, 1990). These reagents have been shown to both inhibit and stimulate a number of physiological responses related to TNF action. For example in Engelman et al, rabbit polyclonal antibodies raised against the soluble form of TNF receptor are cytotoxic to TNF-sensitive cells, are mitogenic to fibroblasts, and induce prostaglandin synthesis. However, Fab fragments of such antibodies are incapable of these TNF-like activities until cross-linked. Rather than mimicking TNF, the antibodies of Shalaby et al behave as TNF inhibitors. In particular the antibodies inhibit TNF cytotoxicity, inhibit TNF-dependent IL6 production and inhibit TNF-induced increases in cell adhesiveness.

Because the unique abilities of the immune system allow molecular complementation of protein structure, the immune response to an anti-ligand antibody can produce an antibody which mimics the biological activity of the original ligand antigen. Such antibodies are known as anti-idiotypic antibodies and they have been used to identify receptors, act as receptor antagonists or agonists, and may also be useful as vaccines (extensively reviewed in Kohler et al, Methods of Enzymology. 178: 3:35, 1989). One of the first anti-idiotypic antibodies was raised against an anti-insulin receptor antibody and had insulin-like activity (Sege and Peterson, Proc. Natl. Acad. Sci, USA. 75: 2443-2447, 1978). A murine anti-idiotypic antibody with the cytokine activity of interferon a has been produced (Osheroff et al, J. Immunol. 135: 306-313, 1985). Anti-substance P anti-idiotypic antibodies behaved as either substance P agonists and antagonists depending on the biological test (Couraud et al, J. Biol. Chem. 260: 9461-9469, 1985). We have found it is possible to prepare anti-idiotypic antibodies which mimic TNF activity. Details of this discovery are described below.

### Summary of the Invention

The antibody of this disclosure is an anti- idiotypic antibody that binds to the TNF binding site of a TNF receptor on a cell surface. Specifically, the antibody will bind to the 55 kd and 75 kd TNF receptor and can be used to mimic TNF activity, as a TNF agonist or antagonist. In one embodiment, the antibody kills more than 50% of WEHI 164 cells at a concentration of 10 ng/ml antibody. WEHI 164 cells are sensitive to the cytotoxic activity of TNF (Espevik and Nissen-Meyer. J. Immunol. Methods 95: 99-105, 1986). The antibody may be made to be non-immunogenic in a given species of animal, including humans. As used herein, the expression mimics TNF activity means production of a physiological response which is similar to the response by TNF. Examples of TNF-induced responses are killing of TNF-sensitive cells, alterations in lipid metabolisms, fibroblast proliferation, and specific gene induction.

The antibody may be made by injecting TNF into one species of animal to generate anti-TNF antibodies having a TNF binding site, harvesting the antibodies, using the harvested antibodies as immunogens to generate other antibodies to the TNF binding site of the harvested antibodies and then harvesting the other antibodies.

The antibodies may be used therapeutically (in a pharmaceutically acceptable carrier such as a buffered agueous solution) to accomplish a given result (e.g. acting as a TNF agonist or antagonist) by administering the antibodies to a mammal under conditions sufficient to accomplish the result.

### Brief Description of the Figures

Figure 1 is a graph illustrating how the antibodies of this disclosure caused toxicity of mouse WEHI 164 cells.
Figure 2 is a graph illustrating how the activity of the antibodies of this disclosure is neutralized by anti-TNF.

### Specific Embodiments

This invention makes use of anti-idiotypic antibody technology and the known function of TNF receptors to produce a unique reagent which may itself be therapeutically useful, and furthermore can also be used to produce other therapeutically useful antibodies. The predominant advantage of this reagent is that it must, by definition and derivation, interact with the TNF binding site on TNF receptors, and not with adjacent sites.

The anti-idiotypic antibody which has TNF-like activity has a portion which is thought to be a molecular imitation of that portion of TNF which binds to a TNF receptor. However, unlike TNF, the antibody can be produced in the extremely large quantities that would be needed for human therapy by fermentation technology, can be derivatized with various biological modifiers to alter its action (e.g. toxins, radioactive nuclides, and even portions of other molecules), and itself can be altered for different functional purposes (e.g. made into Tab fragments, conjugated or aggregated into multivalent complexes, and chimerized with human constant region determinants). Additionally, the immunogen used to produce the anti-idiotypic antibody (FT2 in the example below) is itself an antibody (A10G10 in the example below) and unlike TNF receptors can be produced in large quantities.

The approach of making anti-TNF receptor antibodies from existing anti-TNF antibodies obviates the possibility of obtaining antibodies which produce effects by steric hindrance rather than true binding to the ligand binding domain of TNF receptor. An antibody which is a true molecular imitation of TNF should inhibit TNF binding, by making an exact fit with the receptor binding site for TNF. Other antibodies which inhibit TNF binding may do so only by steric interactions with adjacent sites. Antibodies which stimulate TNF receptor action may crosslink receptors, but not be directed to the ligand binding site on the receptor. Because TNF receptor action seems to require receptor crosslinking, an anti-idiotypic antibody which mimics TNF will inhibit or stimulate depending on the antibody form, i.e. whether a monovalent Tab or a bivalent intact IgG.

It is intended that the antibodies of this disclosure include both intact antibodies and antibody fragments (e.g. to Tab) that retain the portion of the anti-idiotypic antibody which is a molecular imitation of TNF. It is expected that antibody fragments which inhibit TNF binding, but are unable to crosslink receptors will act as TNF inhibitors. Depending on the degree to which intact anti-idiotypic antibodies crosslink TNF receptors, intact antibodies will either stimulate or inhibit TNF receptor action.

By extension of this invention, it is also apparent that it may be possible to produce various species specific monoclonal anti-idiotypic antibodies and even human anti-idiotypic antibodies which would not be expected to be immunogenic in humans. The existence of an anti-idiotypic antibody which mimics TNF makes it possible to generate a new class of antibody reagents which may interact with either TNF receptor or with TNF. For example, it should be apparent to those skilled in the art that the same anti-TNF antibody used to generate one type of anti-idiotypic antibody (e.g. FT2, below) could be used to immunise rats and produce monoclonal anti-idiotypic antibodies of other species (e.g. rats) which are also molecular imitations of TNF. Such a monoclonal antibody could be chimerized with human constant region determinants, making it less immunogenic as a therapeutic antibody.

Similarly, any human patient receiving an anti-TNF antibody may also produce anti-idiotypic antibodies which mimic TNF (for example see Durrant et al, Cancer Immuno. and Immunother. 31: 226-30, 1990). B cells from patients such as these may produce a human antibody which mimics TNF action. Such an antibody would be obtained by immortalizing human antibody-producing cells with Epstein-Barr virus or by cell fusion with suitable fusion partners (see for example Taller et al, Br. J. Cancer. 62: 595-8, 1990 and Gorny et al, Proc. Natl. Acad. Sci, USA. 88: 3238-3242, 1991). Such a human antibody would not be immunogenic in humans and could be used for therapy in humans. Human anti-TNF receptor antibodies could also be obtained by in vitro immunization using anti-TNF as an antigen to stimulate human cells (for an example using mouse cells see Vaux et al, Nature. 336: 36-41, 1988 and Vaux et al, Nature. 345: 495-502, 1990).

Because the anti-idiotypic antibody which mimics TNF has a portion which is a molecular imitation of a portion of TNF, an antibody produced in response to immunization with that antibody should function as an anti-TNF antibody. The antiidiotypic antibody could also be used for in vitro immunization of human cells. A human antibody directed against TNF would not be expected to be immunogenic in humans, and could most likely be administered in multiple dosages over time. Such a human anti-TNF antibody may be very useful to treat not only septic shock, but also chronic conditions marked by excessive immune stimulation like that found in organ transplantation, arthritis and other autoimmune diseases.

Because the anti-idiotypic antibody which mimics TNF is a molecular imitation of TNF, molecular analysis of the structure of the antibody may lead to development of other drugs which are TNF agonists or antagonists. The antibodies of this disclosure contain the molecular information necessary to activate TNF receptors. Detailed analysis of the exact site on the antibody which interacts with TNF receptors may allow one to design other molecules (not necessarily antibodies) which interact with TNF receptors. This approach to drug design is being used to help design drugs for use in acquired immune deficiency (Casale et al, J. Mol. Biol. 216: 511-2, 1990 and Felgenhauer et al, Nucl. Acids. Res. 18: 4927, 1990).

### MATERIALS AND METHODS

Rabbit immunisations: New Zealand white rabbits were injected intradermally with anti-TNT (A10G10), ATCC Accession No. HB9736, in complete Freund's adjuvant. After one month, the rabbits were boosted intramuscularly. The rabbits were repeatedly boosted at irregular intervals with A10G10 over a one year period.

Purification of FT2: Total immunoglobulin was isolated from rabbit serum by precipitation with caprylic acid and ammonium sulfate (J. Immunol. Methods 96: 271-178, 1987). Antibodies directed toward all determinants of A10G10 were isolated from total immunoglobulin by affinity purification over an 8 x 3 cm column of A10G10 coupled to Hydrazide AvidGel F (Bioprobe International) in phosphate buffered saline, 0.05% NaN₃, pH 7.2 buffer and eluted with 0.1 M glycine, pH 2.5. Eluted antibody was neutralized by diluting tenfold into phosphate-buffered saline and concentrated using an Amicon PM 30 membrane. Anti-isotypic antibodies were removed by absorption to a column of C7F7 (a mouse monoclonal anti-Factor VIII antibody of the same subclass) coupled to Hydrazide AvidGel F. The unbound antibody (designated FT2) was pooled, concentrated, and reapplied to the C7F7 column, then concentrated, dialyzed against PBS, pH 7.2, sterile filtered, and stored at 4 C.

Cytotoxicity and neutralization: The toxicity of FT2 was measured by MTT uptake into WEHI 164 clone 13 cells (Galloway et al, in press J. Immunol. Methods. See also, Espevik and Nissen-Meyer, J. Immunol. Methods 95: 99-105, 1986).

### Example

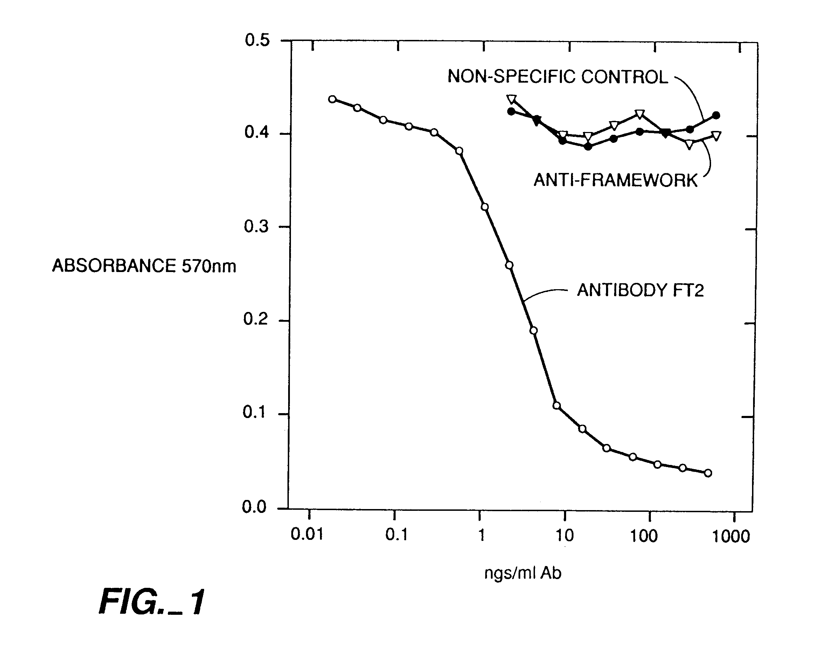

In Fig. 1, mouse WEHI 164 cells were exposed to dilutions of FT2, a rabbit non-specific antibody (directed to a TNF peptide) and an anti-framework antibody also derived by immunizing rabbits against A10G10. An anti-framework antibody (anti-isotypic antibody) is an antibody that also binds the anti-TNF antibody (e.g. A10G10) but does not bind to the region of the anti-TNF that binds to TNF. The anti-framework antibody was purified from the same sera as FT2, but was recovered by elution from the C7F7-affinity column with low pH. FT2 does not bind to the C7F7 affinity column (see methods). After overnight at 37 C, the cell survival was measured by uptake of MTT, a thiazol dye (Galloway et al, in press J. Immunol. Methods. See also Mosmann, J. Immunol. Methods 65: 55-63, 1983.). As can be seen in Fig. 1, incubation of cells with antibody FT2 causes toxicity, while incubation of cells with either a non-specific control or the anti-framework antibody caused no toxicity. In fact, at less than 10 ng/ml FT2, greater than 50% of the cells are killed. Antibodies directed to framework regions of A10G10 do not have this activity.

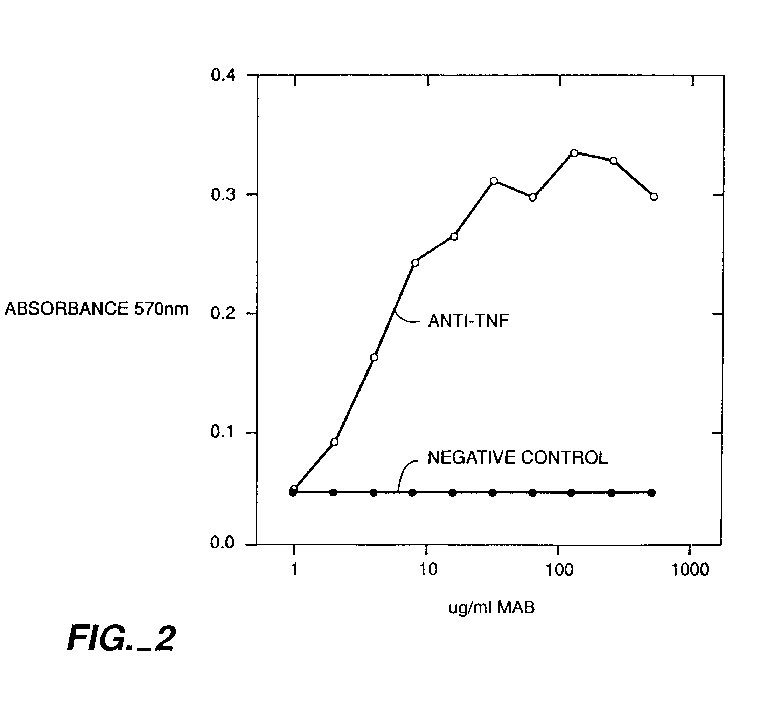

To determine that the toxic activity of this antibody was indeed due to antibody and not to some contaminant, A10G10 was used to neutralize the toxic activity of FT2. In Fig. 2, WEHI 164 cells were incubated with a constant concentration of 1 u.g/ml FT2. Anti-TNF (A10G10) effectively neutralized the toxic activity of FT2. The negative control antibody (murine monoclonal anti-Factor VIII) had no effect. Therefore, the TNF-like toxic activity of FT2 was caused by an antibody and not an exogenous contaminant.

It is thought that the anti-idiotypic antibodies of this disclosure may have diagnostic uses as well as therapeutic uses. For example, soluble TNT receptors have been found in body fluids (e.g. urine) and the concentration of these receptors may be correlated with progress of a disease by using immunoassay techniques.

The above example should be considered illustrative only and it is intended that the scope of this disclosure should be limited only by the following claims.

## Claims

1. An antibody that specifically binds to the TNF binding site of a TNF receptor and mimics TNF activity.

2. The antibody of claim 1 wherein the antibody binds to both the 55 kd and 75 kd TNF receptor.

3. The antibody of claim 1 having the characteristic of killing more than 50% of WEHI 164 cells at 10 ng/ml antibody.

4. The antibody of claim 1 which binds with the TNF binding site of the antibody A10G10 expressed from the cell line having ATCC Accession No. HB9736.

5. The antibody of claim 1 in a pharmaceutically acceptable carrier.

6. A method of making an antibody that binds to the TNF binding site of a TNF receptor, the method comprising the steps of
(a) injecting TNF in a mammal to generate anti-TNF antibodies having a TNF binding site;
(b) harvesting the anti-TNF antibodies of step (a);
(c) using the harvested antibodies to generate other antibodies to the TNF binding site of the harvested antibodies; and
(d) harvesting the other antibodies of step (c).

7. A method of mimicking the effect of TNF to accomplish a given result comprising administering to a mammal antibodies that bind to TNF receptors found in the mammal, the administration being under conditions sufficient to accomplish the result.

8. The method of claim 7 wherein the given result is controlling the availability of TNF receptor sites.

9. The method of claim 7 wherein the given result is crosslinking TNF receptor sites.

10. The method of claim 7 wherein the given result is controlling the amount of TNF receptor found in bodily fluids.

11. The method of claim 7 wherein the given result is causing the death of TNF-sensitive cells.

12. The method of claim 7 wherein the given result is to stimulate or inhibit TNF-dependent immune responses.

13. The method of claim 7 wherein the antibodies are used therapeutically.
